(19) **Europäisches Patentamt / European Patent Office / Office européen des brevets**

(11) **EP 1 173 480 B1**

(12) **EUROPEAN PATENT SPECIFICATION**

(45) Date of publication and mention of the grant of the patent:
**16.01.2008 Bulletin 2008/03**

(21) Application number: **00926599.2**

(22) Date of filing: **04.05.2000**

(51) Int Cl.:
*C07K 14/47* (2006.01)　　*A61K 38/17* (2006.01)
*G01N 33/68* (2006.01)　　*A61P 25/28* (2006.01)
*C12N 5/00* (2006.01)　　*A61K 51/00* (2006.01)
*C07K 5/10* (2006.01)

(86) International application number:
**PCT/CA2000/000515**

(87) International publication number:
**WO 2000/068263 (16.11.2000 Gazette 2000/46)**

(54) **STEREOSELECTIVE ANTIFIBRILLOGENIC PEPTIDES**

STEREOSELEKTIVE ANTIFIBRILLOGENE PEPTIDE

PEPTIDES STEREOSELECTIFS ANTIFIBRILLOGENES

(84) Designated Contracting States:
**AT BE CH CY DE DK ES FI FR GB GR IE IT LI LU MC NL PT SE**

(30) Priority: **05.05.1999 US 132592 P**

(43) Date of publication of application:
**23.01.2002 Bulletin 2002/04**

(73) Proprietor: **Neurochem (International) Limited 1015 Lausanne (Ecublens) (CH)**

(72) Inventors:
- **CHALIFOUR, Robert**
  **Ile Bizard, Québec H9C 1T4 (CA)**
- **GERVAIS, Francine**
  **Ile Bizard, Québec H9C 3A9 (CA)**
- **GUPTA, Ajay**
  **Pointe-Claire, Québec H9R 5L6 (CA)**

(74) Representative: **Evans, Claire et al Marks & Clerk Incorporating Edward Evans Barker 90 Long Acre London WC2E 9RA (GB)**

(56) References cited:
**WO-A-96/28471　　WO-A-97/21728
WO-A-98/08868**

- TJERNBERG L O ET AL: "CONTROLLING AMYLOID BETA-PEPTIDE FIBRIL FORMATION WITH PROTEASE-STABLE LIGANDS" JOURNAL OF BIOLOGICAL CHEMISTRY,US,AMERICAN SOCIETY OF BIOLOGICAL CHEMISTS, BALTIMORE, MD, vol. 272, no. 19, 9 May 1997 (1997-05-09), pages 12601-12605, XP002050230 ISSN: 0021-9258 cited in the application
- GIULIAN E.A.: "The HHQK domain of beta-amyloid provides a structural basis for the immunopathology of Alzheimer disease" JOURNAL OF BIOLOGICAL CHEMISTRY, vol. 273, no. 45, 6 November 1998 (1998-11-06), pages 29719-29726, XP002146049 MD US cited in the application
- DATABASE WPI Section Ch, Week 199837 Derwent Publications Ltd., London, GB; Class B04, AN 1998-433888 XP002154640 -& JP 10 182695 A (TEIKOKU SEIYAKU KK), 7 July 1998 (1998-07-07)
- KOERNYEI J ET AL: "TC-99M LABELLING AND BIODISTRIBUTION OF DESIGNED MOLECULES" RADIOACTIVE ISOTOPES IN CLINICAL MEDICINE AND RESEARCH,BIRKHAEUSER VERLAG, BASEL,CH, 1995, pages 287-292, XP000965479
- TORNEIRO E.A.: "Sequence-elective binding of pepides in water by a synthetic receptor molecule" JOURNAL OF THE AMERICAN CHEMICAL SOCIETY, vol. 117, no. 21, 1995, pages 5887-5888, XP002154639 DC US

SEQUENCE LISTING

<110> NEUROCHEM INC.
CHALIFOUR, Robert
GERVAIS, Francine
GUPTA, Ajay

<120> STEREOSELECTIVE ANTIFIBRILLOGENIC
PEPTIDES AND PEPTIDOMIMETICS THEREOF

<130> 14228-1PCT

<150> US 60/132,592
<151> 1999-05-05

<160> 24

<170> FastSEQ for Windows Version 3.0

<210> 1
<211> 6
<212> PRT
<213> Artificial Sequence

<220>
<223> peptide having antifibrillogenic activity and/or cytoprotection

<400> 1

```
        Lys Ile Val Phe Phe Ala
         1               5
```

<210> 2
<211> 7
<212> PRT
<213> Artificial Sequence

<220>
<223> peptide having antifibrillogenic activity and/or cytoprotection

<400> 2

```
        Lys Lys Leu Val Phe Phe Ala
         1               5
```

<210> 3
<211> 6
<212> PRT
<213> Artificial Sequence

<220>
<223> peptide having antifibrillogenic activity and/or cytoprotection

<400> 3

```
        Lys Leu Val Phe Phe Ala


            1               5
```

<210> 4
<211> 6
<212> PRT
<213> Artificial Sequence

<220>
<223> peptide having antifibrillogenic activity and/or cytoprotection

<400> 4

```
Lys Phe Val Phe Phe Ala
 1               5
```

<210> 5
<211> 6
<212> PRT
<213> Artificial Sequence

<220>
<223> peptide having antifibrillogenic activity and/or cytoprotection

<400> 5

```
Ala Phe Phe Val Leu Lys
 1               5
```

<210> 6
<211> 4
<212> PRT
<213> Artificial Sequence

<220>
<223> peptide having antifibrillogenic activity and/or cytoprotection

<400> 6

```
Lys Leu Val Phe
 1
```

<210> 7
<211> 6
<212> PRT
<213> Artificial Sequence

<220>
<223> peptide having antifibrillogenic activity and/or cytoprotection

<400> 7

```
Lys Ala Val Phe Phe Ala
 1               5
```

<210> 8
<211> 5
<212> PRT
<213> Artificial Sequence

<220>
<223> peptide having antifibrillogenic activity and/or cytoprotection

<400> 8

```
      Lys Leu Val Phe Phe
      1               5
```

<210> 9
<211> 6
<212> PRT
<213> Artificial Sequence

<220>
<223> peptide having antifibrillogenic activity and/or cytoprotection

<400> 9

```
      Lys Val Val Phe Phe Ala
      1               5
```

<210> 10
<211> 6
<212> PRT
<213> Artificial Sequence

<220>
<223> peptide having antifibrillogenic activity and/or cytoprotection

<221> AMIDATION
<222> (6) ... (6)

<400> 10

```
      Lys Ile Val Phe Phe Ala
      1               5
```

<210> 11
<211> 6
<212> PRT
<213> Artificial Sequence

<220>
<223> peptide having antifibrillogenic activity and/or cytoprotection

<221> AMIDATION
<222> (6)...(6)

<400> 11

```
      Lys Leu Val Phe Phe Ala
      1               5
```

<210> 12
<211> 6
<212> PRT
<213> Artificial Sequence

<220>

<223> peptide having antifibrillogenic activity and/or cytoprotection

<221> AMIDATION
<222> (6) ... (6)

<400> 12

```
        Lys Phe Val Phe Phe Ala
         1               5
```

<210> 13
<211> 6
<212> PRT
<213> Artificial Sequence

<220>
<223> peptide having antifibrillogenic activity and/or cytoprotection

<221> AMIDATION
<222> (6)...(6)

<400> 13

```
        Ala Phe Phe Val Leu Lys
         1               5
```

<210> 14
<211> 4
<212> PRT
<213> Artificial Sequence

<220>
<223> peptide having antifibrillogenic activity and/or cytoprotection

<221> AMIDATION
<222> (4) ... (4)

<400> 14

```
        Lys Leu Val Phe
         1
```

<210> 15
<211> 6
<212> PRT
<213> Artificial Sequence

<220>
<223> peptide having antifibrillogenic activity and/or cytoprotection

<221> AMIDATION
<222> (6) ... (6)

<400> 15

```
        Lys Ala Val Phe Phe Ala
         1               5
```

<210> 16
<211> 5
<212> PRT
<213> Artificial Sequence

<220>
<223> peptide having antifibrillogenic activity and/or cytoprotection

<221> AMIDATION
<222> (5)...(5)

<400> 16

```
        Lys Leu Val Phe Phe
         1               5
```

<210> 17
<211> 6
<212> PRT
<213> Artificial Sequence

<220>
<223> peptide having antifibrillogenic activity and/or cytoprotection

<221> AMIDATION
<222> (6)...(6)

<400> 17

```
        Lys Val Val Phe Phe Ala
         1               5
```

<210> 18
<211> 7
<212> PRT
<213> Artificial Sequence

<220>
<223> peptide having antifibrillogenic activity and/or cytoprotection

<400> 18

```
        Lys Leu Val Phe Phe Ala Gln
         1               5
```

<210> 19
<211> 7
<212> PRT
<213> Artificial Sequence

<220>

<223> peptide having antifibrillogenic activity and/or cytoprotection

<221> AMIDATION
<222> (7) ... (7)

<400> 19

```
    Lys Leu Val Phe Phe Ala Gln
     1               5
```

<210> 20
<211> 9
<212> PRT
<213> Artificial Sequence

<220>
<223> peptide having antifibrillogenic activity and/or cytoprotection

<221> AMIDATION
<222> (9)...(9)

<400> 20

```
    His His Gln Lys Leu Val Phe Phe Ala
     1               5
```

<210> 21
<211> 3
<212> PRT
<213> Artificial Sequence

<220>
<223> peptide having antifibrillogenic activity and/or cytoprotection

<400> 21

```
    Asp Asp Asp
     1
```

<210> 22
<211> 6
<212> PRT
<213> Artificial Sequence

<220>
<223> peptide having antifibrillogenic activity and/or cytoprotection

<400> 22

```
    Lys Val Asp Asp Gln Asp
     1               5
```

<210> 23

<211> 4
<212> PRT
<213> Artificial Sequence

<220>
<223> peptide having antifibrillogenic activity and/or cytoprotection

<400> 23

```
His His Gln Lys
  1
```

<210> 24
<211> 6
<212> PRT
<213> Artificial Sequence

<220>
<223> peptide having antifibrillogenic activity and/or cytoprotection

<221> AMIDATION
<222> (6)...(6)

<400> 24

```
Gln Lys Leu Val Phe Phe
  1               5
```

**Description**

## BACKGROUND OF THE INVENTION

(a) Field of the Invention

The invention relates to agents having potent antifibrillogenic activity for the treatment of amyloidosis disorders and for imaging of amyloid deposits. These agents include peptides and peptidomimetic compounds thereof.

(b) Description of Prior Art

Amyloidosis refers to a pathological condition characterized by the presence of amyloid fibers. Amyloid is a generic term referring to a group of diverse but specific extracellular protein deposits that are seen in a number of different diseases. Though diverse in their occurrence, all amyloid deposits share common morphologic properties, stain with specific dyes (e.g. Congo red), and have a characteristic red-green birefringent appearance in polarized light after staining. They also share common ultrastructural, x-ray diffraction and infrared spectra features.

Some amyloidotic diseases can be idiopathic but most of these diseases appear as a complication of a previously existing disorder. For example, primary amyloidosis can appear without any other pathology or can follow plasma cell dyscrasia or multiple myeloma. Secondary amyloidosis is usually seen associated with chronic infection (such as tuberculosis) or chronic inflammation (such as rheumatoid arthritis). A familial form of secondary amyloidosis is also seen in Familial Mediterranean Fever (FMF). This familial type of amyloidosis, as one of the other types of familial amyloidosis, is genetically inherited and is found in specific population groups. Isolated forms of amyloidosis are those that tend to involve a single organ system. Different amyloids are also characterized by the type of protein present in the deposit. For example, neurodegenerative diseases such as scrapie, bovine spongiform encephalitis, Creutzfeldt-Jakob disease and the like are characterized by the appearance and accumulation of a protease-resistant form of a prion protein (referred to as AScr or PrP-27) in the central nervous system. Similarly, Alzheimer's disease, another neurodegenerative disorder, is characterized by congophilic cerebral angiopathy, neuritic plaques and neurofibrillary tangles. In this case, the plaque and blood vessel amyloid is formed by the deposition of fibrillar Aβ amyloid protein. In adult-onset diabetes, amyloids containing the IAPP amyloid protein accumulate in the pancreas. Other systemic diseases, complications of long-term hemodialysis and sequelae of long-standing inflammation or plasma cell dyscrasias are characterized by the accumulation of amyloids systemically. In each of these cases, a different amyloidogenic protein is involved in amyloid deposition.

Once these amyloids have formed, there is no known, widely accepted therapy or treatment that significantly dissolves the deposits in situ.

Each amyloidogenic protein has the ability to organize into β-sheet and to form insoluble fibrils that get deposited extracellularly. Each amyloidogenic protein, although different in amino acid sequence has the same property of forming fibrils and binding to other elements such as proteoglycan (glycosaminoglycan), amyloid P and complement component. Moreover, each amyloidogenic protein has amino acid sequences which, although different, will show similarities such as regions with the ability to bind to GAG's (referred to as the GAG binding site) as well as other regions which will promote β-sheet formation referred to as β-sheet region.

In specific cases, amyloidotic fibrils once deposited can become toxic to the surrounding cells. As per example, the Aβ fibrils organized as senile plaques have been shown to be associated with dead neuronal cells and microgliosis in patients with Alzheimer's disease. When tested in vitro, Aβ peptide was shown to be capable of triggering an activation process of the microglia (brain macrophages), which would explain the presence of microgliosis and brain inflammation found in the brain of patients with Alzheimer's disease.

In another type of amyloidosis seen in patients with Type II diabetes, the amyloidogenic protein IAPP, has been shown to induce β-islet cell toxicity *in vitro,* Hence, appearance of IAPP fibrils in the pancreas of Type II diabetic patients could contribute to the loss of the β islet cells (Langerhans) and organ dysfunction.

Particularly, in patients with Alzheimer's Disease, an agent capable 1) of preventing amyloid fibril formation and deposition and 2) of directly or indirectly inhibiting Aβ-induced neurotoxicity and inflammation (microgliosis), could be a treatment of choice to prevent and arrest the development of Alzheimer's disease.

WO-A-9808868 concerns compounds that modulate natural beta-amyloid peptide aggregation. The compounds comprise a peptide, preferably based on a beta -amyloid peptide, that is comprised of 3-5 D-amino acid residues and includes at least two D-amino acid residues independently selected from the group consisting of D-leucine, D-phenylalanine and .D-valine. In one embodiment the peptide is a retro-inverso isomer of a beta -amyloid peptide. In certain embodiments, the peptide is modified at the amino-terminus, the carboxy-terminus, or both.

It would be highly desirable to be provided with agents having potent antifibrillogenic activity for the treatment of amyloidosis disorders.

Relevant prior art includes Tjernberg L.O. et al (Journal Biol.Chem. Vol. 272, No. 19, 1997, 12601-12605).

## SUMMARY OF THE INVENTION

One aim of the present invention is to provide agents having potent antifibrillogenic activity for the treatment of amyloidosis disorders.

Another aim of the present invention is to provide a method for the treatment of amyloidosis disorders, such as Alzheimer's' disease.

A number of strategies for possible therapeutic intervention in amyloid development have been proposed. These strategies include reduction of the pool of precursor proteins, prevention of the interaction of precursor proteins and disruption of preformed amyloid. The present invention deals mainly with the second approach, prevention of precursor protein interactions. The ideal molecule to fulfill this function, would interact specifically with the amyloid protein and would in so doing prevent the protein from interacting with itself. When dealing with molecules that are chiral, it is standard practice to identify which of the stereoisomers possesses the activity, since in general, activity can be attributed to one or the other of the isomers. By using a stereochemically pure isomer, side reactions can be avoided or reduced.

In accordance with one embodiment of the present invention there is provided an antifibrillogenic agent for inhibiting amyloidosis and/or for cytoprotection, which comprises a peptide consisting of Formula I and consisting of all [D]-amino acids, or a retro-isomer of a peptide consisting of Formula I and consisting of all [D]-amino acids:

$$Xaa_1\text{-}Xaa_2\text{-}Xaa_3\text{-}Xaa_4 \qquad \text{(Formula I)}$$

wherein,

$Xaa_1$ is selected from the group consisting of Lys and $Xaa_5$-Lys-;
$Xaa_5$ is selected from the group consisting of Lys, His-Gln-, His-His-Gln-, Val-His-His-Gln-, Glu-Val-His-His-Gln-, Asp-Asp-Asp-, and Gln-;
$Xaa_2$ is any amino acid;
$Xaa_3$ is Val; and
$Xaa_4$ is selected from the group consisting of Phe, Phe-$NH_2$, Phe-Phe, Phe-Phe-$NH_2$, Phe-Phe-Ala, Phe-Phe-Ala-$NH_2$, Phe-Phe-Ala-Gln, and Phe-Phe-Ala-Gln-$NH_2$.

Preferably, $X_{aa2}$ is an amino acid residue selected from the group consisting of Leu, Ile, Ala, Val and Phe.

Preferably, $Xaa_2$, is a hydrophobic amino acid residue such as a leucine residue.

Preferably, the retro-isomer is selected from the group consisting of SEQ ID NOs: 5 and 13.

Preferably, the peptide of Formula I is selected from the group consisting of SEQ ID Nos: 1-4, 6-12, 14-20, and 24.

More preferably, the peptide of formula I is a peptide as set forth in SEQ ID NO: 2 or SEQ ID NO: 3.

The antifibrillogenic agent is preferably formulated in a composition comprising a therapeutically effective amount of said antifibrillogenic agent and a pharmaceutically effective carrier.

Also provided according to the present invention is the antifibrillogenic agent of the present invention, wherein said peptide of Formula I or retro-isomer thereof is conjugated to a label for *in vivo* imaging.

Preferably, the label is $^{99m}Tc$.

Also provided is the conjugated peptide or retro-isomer for use in the manufacture of a compound for in vivo imaging of amyloid deposit in a mammal

In accordance with one embodiment of the present invention there is provided a labelled conjugate for in vivo imaging of amyloid plaque, which comprises a conjugate of formula II:

$$A\text{-}B\text{-}C \qquad II$$

wherein A is an amyloid plaque-targeting moiety selected from the group consisting of a peptide of Formula I as defined above, and a retro isomer thereof,
wherein B is a linker portion allowing attachment of the amyloid plaque-targeting moiety to C; and
wherein C is a label that allows for in vivo imaging.

Preferably, the linker portion B is selected from the group consisting of Glucose and Phe. Preferably, the label C is $^{99m}Tc$.

Also provided according to the present invention is the antifibrillogenic agent of the present invention, for use in the treatment of amyloid-β disorders I a patent.

Also provided is the use of an antifibrillogenic agent according to the present invention in the manufacture of a medicament for the treatment of amyloid-β disorders in a patient.

The medicament preferably comprises a therapeutically effective amount of a peptide of Formula I or a retro-isomer thereof, and a pharmaceutically acceptable carrier.

Preferably, the amyloid-β disorder is a neurodegenerative disorder, such as Alzheimer's disease.

Diseases caused by the death or malfunctioning of a particular type or types of cells can be treated by transplanting into the patient healthy cells of the relevant type of cell. Often these cells are cultured in vitro prior to transplantation to increase their numbers, to allow them to recover after the isolation procedure or to reduce their immuogenicity. However, in many instances the transplants are unsuccessful, due to the death of the transplanted cells. The inventors have now also found that culturing of cells can lead to the formation of fibrils from endogenous proteins. Such fibrils are likely to continue to grow after the cells are transplanted and cause death or dysfunction of the cells. The inventors have also found that the peptide of the present invention or the antifibrillogenic compound of the present invention can be used to reduce the formation of fibrils.

The peptide of Formula I or the antifibrillogenic compound causes breakdown of amyloid deposits which have been formed by the cells prior to the contact. Preferably, the cells are cultured in the presence of the peptide of Formula I or the antifibrillogenic compound.

For the purpose of the present invention, the following expressions and terms are defined below.

The term "agents having stereoselective antifibrillogenic activity" is intended to mean any peptides, peptide analogues, peptide derivatives, or peptidomimetics which retain the stereoselective antifibrillogenic activity, the cytoprotective and anti-inflammatory activity and/or the ability to alter a natural amyloidotic protein aggregation as described herein. Peptide analogues, peptide derivatives, or peptidomimetics include any molecules that mimic the chemical structure of a peptide and retain the functional properties of the peptide (Williams, W.V. and Weiner, D.B., eds., Biologically Active Peptides: Design, Synthesis, and Utilization, vol. 1, Technomic Publishing Company Inc., Lancaster, Pa., 1993, pages 35-3..). Examples of peptide analogues, peptide derivatives, or peptidomimetics include compounds with sulfonamide, phosphoramide or non-amide linkages.

The expression "antifibrillogenic activity" is intended to mean the ability to block or prevent an amyloidogenic protein from forming fibrils, preferably by preventing it from adopting its β-pleated conformation.

The term "cytoprotection" or "cytoprotective activity" is intended to mean the ability to protect cells from amyloid-induced toxicity.

The expression "anti-inflammatory" is intended to mean the ability to block or reduce the Aβ-induced microglial activation process or to block the chemokine-induced inflammatory reaction.

The expression "retro isomer" is intended to mean a reversal of the direction of the peptide backbone.

The expression "inverso isomer" is intended to mean an inversion of the amino acid chirality used to make the peptide.

The expression "retro-inverso isomer" is intended to mean a reversal of both the peptide backbone direction and the amino acid chirality.

Except as otherwise expressly defined herein, the abbreviations used herein for designating the amino acids and the protective groups are based on recommendations of the IUPAC-IUB Commission on Biochemical Nomenclature (Biochemistry, 1972, 11:1726-1732).

Also, unless specified otherwise, the Aβ(1-40) is the naturally occurring Aβ(1-40), that is the all [L] -isomer.

## BRIEF DESCRIPTION OF THE DRAWINGS

Fig. 1 illustrates the targeted sites of the protein-protein interactions required for self-assembly into β-sheet fibrils;

Fig. 2 illustrates a thioflavin T fluorescence assay for fibril formation by [L]-Aβ (1-40) in the absence and presence of a peptide in accordance with one embodiment of the invention;

Fig. 3 shows the same assay as in Fig. 2 for fibril formation by [D]-Aβ (1-40);

Fig. 4 is a bar graph illustrating the percentage of thioflavin T fluorescence in the presence of the [D]-peptide used in Fig. 2, with or without single substitutions of corresponding [L]-amino acids;

Fig. 5 is a bar graph illustrating a thioflavin T fluorescence assay for fibril formation by [L]-Aβ (1-40) in the presence of the [D]-peptide used in Fig. 2, with or without substitution of the Leu residue by other hydrophobic amino acids;

Fig. 6 illustrates the toxicity of [L] -Aβ (1-40) in the absence and presence of peptides in accordance with one embodiment of the invention; and

Fig. 7 is a bar graph illustrating the toxicity of [L]-Aβ (1-40) in the presence of another peptide of the present invention.

## DETAILED DESCRIPTION OF THE INVENTION

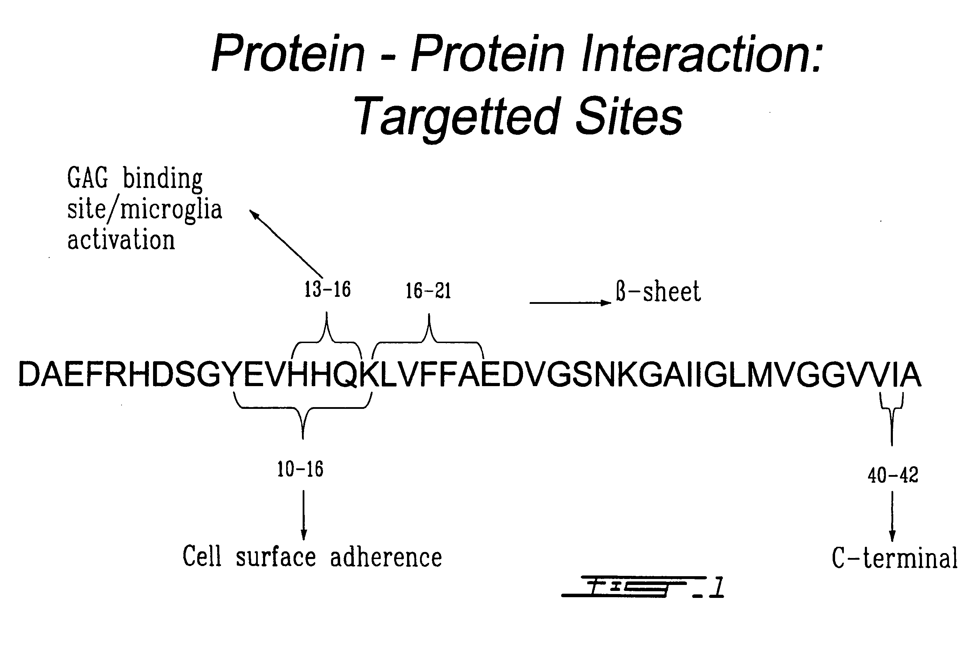

As illustrated in Fig. 1, internal regions of the Aβ sequence have been shown to confer characteristics of the amyloid protein. Indeed, the region between amino acid 13-16 (His-His-Gln-Lys, SEQ ID NO:23) of the amyloid protein is responsible for the interaction between the Aβ protein and the glycosaminoglycan moiety of the proteoglycans (Kisilevsky, R., et al., Proteoglycans and amyloid fibrillogenesis: The nature and origin of amyloid fibrils, Wiley, Chichester (CIBA Foundation Symposium 1997), pp. 58-72). Proteoglycans are known to promote amyloid fibril formation as well as protect these fibrils from proteolysis (Gupta-Bansal, R., et al., 1995, The Journal of Biological Chemistry, 270: 18666-18671). More recently, the same region has been determined to play a role in the activation process of microglial cells by Aβ (Giulian, D., et al., 1998, The Journal of Biological Chemistry, 273(45):29719-29726). This 13-16 region of Aβ, often referred to as the GAG binding site, is also part of a larger domain, the 10-16 region of the protein which has been suggested as the region responsible for the adherence of Aβ to the cell surface (Giulian, D., et al., 1996, The Journal of Neuroscience, 16(19):6021-6037). Such adherence of Aβ to the cell surface will allow the interaction of Aβ with the specific cells leading to either microglia activation or toxicity of neuronal cells.

These two overlapping regions of the Aβ protein, i.e. amino acids 13-16 and 10-16 are adjacent to the 16-21 region of Aβ, a short hydrophobic stretch critical for the formation of fibrillar structures (Hilbrich, C., et al., 1992, J. Mol. Biol., 228:460-473). By having peptides capable of interacting with these overlapping regions of Aβ, one can aim at preventing both Aβ fibril formation and Aβ cellular interaction (i.e. microglia activation, neurotoxicity).

A preferred embodiment of the present invention is novel and arises from the unexpected finding that the all-[D] stereoisomer peptides, Lys-Lys-Leu-Val-Phe-Phe-Ala (SEQ ID NO:2) and Lys-Leu-Val-Phe-Phe-Ala (SEQ ID NO:3), are much more potent inhibitors of Aβ(1-40) fibrillogenesis then the corresponding all-[L] peptides. The all-[D] stereoisomer peptides, Lys-Lys-Leu-Val-Phe-Phe-Ala (SEQ ID NO:2) and Lys-Leu-Val-Phe-Phe-Ala (SEQ ID NO:3) are also potent cytoprotective agents.

This finding was unforeseen particularly because the researchers who originally reported peptides containing the sequence Lys-Leu-Val-Phe-Phe-Ala (SEQ ID NO:3) as an inhibitor of fibrillogenesis, state in a second article which they published: "A peptide entirely composed of amino acids in D configuration with the sequence klvff (lowercase marks amino acids in D configuration) was synthesized using the SPOT technique and assayed for $^{125}$I-LBMP1620 binding. This peptide failed to bind $^{125}$I-LBMP1620 indicating that KLVFF-KLVFF interaction is sterospecific." Tjernberg, L.O. et al. (1997) Controlling Amyloid β-Peptide Fibril Formation with Protease-stable Ligands, J. Biol. Chem., 272:12602.

Inhibition of Amyloidosis

The experimental work performed leading to this invention included comparing the ability of the [D] and [L] stereoisomers of peptide Lys-Lys-Leu-Val-Phe-Phe-Ala (SEQ ID NO:2) to inhibit the fibrillogenesis process observed with the amyloidogenic peptide Aβ(1-40) in a thioflavin T fluorescence assay.

The thioflavin T fluorescence assay for fibrillogenesis is based on the principle that the fluorescent dye, thioflavin T, binds specifically to fibrillar, but not to unaggregated Aβ peptide (LeVine III, H., 1993, Protein Science 2:404-410). Upon binding, thioflavin T develops a characteristic fluorescence (Naiki, H., et al., 1996, Lab. Invest. 74: 374-383) which can be easily detected. The dye is believed to interact with the stacked cross-β pleated sheets, the common structural motif of all amyloids (LeVine III, H., 1995, Amyloid: Int. J. Exp. Clin Invest. 2:1.6). Thioflavin T is widely used to assay the effect of compounds on Aβ peptide fibrillogenesis (Bronfman, P.C., et al., 1995, Neuroscience Lett. 218:201-203).

In this assay test compounds are incubated with a solution of Aβ(1-40) (20 μM) containing 10 μM thioflavin T, in 0.02M Tris/0.02M acetate/0.15M NaCl/0.005% azide/pH 7.40 at 37°C in sealed 384 well microplates. Readings (ex 430 nm/em 485nm) are taken at various time intervals with a microplate fluorescence reader. An increase in fluorescence signifies the appearance of amyloid or intermediates in the production of amyloid. Inhibitors of fibrillogenesis will lead to less fluorescence production.

The results illustrated in Table 1 below, are based on the fluorescence production in the presence of test peptides at either 20 μM or 80 μM concentration, at the time intervals of 5, 19, 45, 67, 77 and 90 hours, compared to a control, buffer alone, without added inhibitory peptide.

**Table 1**
**Order Of Potency of Peptide Inhibitors**

|  | Tested at 20μM | Tested at 80μM |
|---|---|---|
| (strongest activity) | 1 (D) KIVFFA | 1 (D) AFFVLK |
|  | 2 (D) KKLVFFA | 1 (D) KKLVFFA |
|  | 3 (D) KLVFFA | 1 (D) KLVFFA |
|  | 4 (D) KFVFFA | 1 (D) KFVFFA |
|  | 5 (D) AFFVLK | 5 (D) KIVFFA |
|  | 6 (D) KLVF | 6 (D) KAVFFA |

(continued)

**Order Of Potency of Peptide Inhibitors**

| | Tested at 20$\mu$M | Tested at 80$\mu$M |
|---|---|---|
| | 7 (D) KAVFFA | 7 (L) KKLVFFA |
| | 8 (L) KLVFFA | 8 (L) KLVFFA |
| | 9 (D) KLVFF | 9 (D) KLVF |
| | 10 (L) KKLVFFA | 10 (D) KLVFF |
| (weakest activity) | 11 (L) AFFVLK | 11 (L) AFFVLK |

## Protocol

A$\beta$ peptide: A$\beta$(1-40) 95% purity (American Peptide Company, Inc, Sunnyvale, Cal. USA, cat. 62-0-78) is disaggregated in trifluoroacetic acid and filtered through a 0.02 $\mu$M filter, (Whatman Anotop 25 plus, .02 $\mu$m, Catalogue no. 6809 4102) in hexafluoroisopropanol (HFIP). Solutions of A$\beta$(1-40) at 600 $\mu$M in HFIP are stored at -80°C.

Assay mixture: The mixture is prepared as two solutions that are combined upon addition to the 384 well microplate (Corning Costar cat. 3705).

i) Solution A consists of test peptides in 0.02M Tris/0.02M acetate/0.15M NaCl/0.01 % azide at pH 7.40 or buffer alone (control),

ii) Solution B consists of A$\beta$(1-40) 40 $\mu$M, thioflavin T 20 $\mu$M in 0.02M Tris/0.02M acetate/0.15M NaCl at pH 7.40. This solution is prepared by drying the A$\beta$ peptide under nitrogen and then resuspending this in 0.04M Tris base with 15 minutes sonication. An equal volume of 0.04M acetic acid containing 0.3 M NaCl is added and the solution is adjusted to pH 7.40$\pm$0.02. A small volume of 5mM thioflavin T is added to the solution to give a final 20 $\mu$M concentration of thioflavin T.

iii) The microplate is loaded with 40 $\mu$L of solution A followed by 40 $\mu$L of solution B which gives a final 20 $\mu$M A$\beta$ (1 -40), 10 $\mu$M thioflavin T, and either 20 $\mu$M, 80 $\mu$M or 100 $\mu$M test compound in 0.02M Tris/0.02M acetate/0.15M NaCl/0.005% azide, pH 7.40. The plate is sealed and loaded into the microplate fluorescence reader.

Fluorescence measurement data analysis: The HTS-7000 Bio Assay Reader, Perkin Elmer, is used to perform kinetic runs of about 5 days. Readings were taken at various time intervals, 5, 19, 45, 67, 77 and 90 hours, with one minute shaking before each reading. Bandpass filters used were: excitation 430 nm, emission 485 mm.

## Calculations

The rank order of efficacy of the peptides is determined by observing which peptides allow the appearance of fluorescence, above the background level, first. For example in the presence of buffer control alone, fluorescence appears earlier than when any of the peptides is present. The most active peptides prevent the appearance of fluorescence even after 90 hours of incubation.

The results achieved in the thioflavin T fibrillogenesis assays show that all-[D] stereoisomer peptide was about 60 times more potent then the all-[L] stereoisomer peptide. This is based on the observation that 400 $\mu$M all-[L] stereoisomer was required to give an equivalent inhibition to that produced with 6.1 $\mu$M all-[D] stereoisomer peptide.

The results achieved in the A$\beta$-NBD environmental probe fibrillogenesis assay showed that the all-[D] stereoisomer peptide was at least 30 times more potent than the all-[L] stereoisomer peptide. This estimate is based on the observation that the lowest concentration of all- [D] peptide tested (25 $\mu$M) was more potent than the highest concentration of the all-[L] peptide (800 $\mu$M).

## $\beta$-sheet and GAG binding domains peptides

Novel peptides and peptidomimetics that include complementary sequences to certain portions of amyloidogenic peptides such as A$\beta$, AA, AL, IAPP, and prion proteins are designed to be capable of inhibition of Protein-Protein interactions or self assembly. The targeted portions in the various disease-causing proteins aforementioned, preferably contain one or more charged residues such as aspartate, glutamate, lysine, histidine and arginine. Such peptides and their peptidomimetics will inhibit fibrillogenesis of the amyloidogenic peptides and prion proteins and interfere with chemokines binding to the cell surface proteoglycans leading to dimerization or tetramerization by interacting with their GAG binding domains. In the case of A$\beta$, these interactions lead to cytoprotection as well as inhibition of inflammatory response and serve as potent therapeutics for the treatment of Alzheimer's disease. In the case of chemokine-related disorders these interactions may lead to a decrease in the uncontrolled inflammatory response associated with some diseases.

Other amyloidogenic peptides such as IAPP, have also been tested. For example, 2 peptides from the β-sheet region of IAPP have been shown to inhibit IAPP fibril formation using the thioflavin T fluorescence assay, circular dichroism (measures secondary structure) and the electron microscope (to look at fibrils directly).

The full-length IAPP is 37 amino acids and the β-sheet region is the 20-29 sequence. The 20-29 sequence is critical for forming β-sheet and has been previously shown to be a key region in modulating IAPP aggregation and folding. Hexapeptides from this β-sheet region were examined and 2 were found to be active.

Hexapeptides spanning the 20-29 region (Ser-Asn-Asn-Phe-Gly-Ala-Ile-Leu-Ser-Ser) of the IAPP protein were synthesized and tested for their ability to prevent fibril formation as determined by circular dichroism and the thioflavin T assay. Hexapeptides were designed and were found to be capable of blocking the formation of IAPP fibrils. These peptides (Ser-Asn-Asn-Phe-Gly-Ala- and Asn-Asn-Phe-Gly-Ala-Ile) were directed towards the central core of the 20-29 region.

Novel peptides containing 3-6 residues that are complementary (in terms of their charges) to the 10-16 segment of Aβ peptide have been shown for the first time to strongly interact with Aβ peptide. They provide a starting point for the design of BBB (blood brain barrier) permeable peptidomimetics. In principle, the present invention provides similar peptides can be designed for the other amyloidogenic peptides such as AA, AL, and IAPP.

Asp-Asp-Asp (SEQ ID NO:21), a tripeptide, when incubated with Aβ40 under physiological conditions shows a slight decrease at time t=0 in the amount of P-sheet content as is evident by the CD spectrum. Incubation of this tripeptide with Aβ40 for 24 hours shows no trace of β-sheet conformation of the Aβ40 and clearly indicates the ability of this tripeptide to strongly interact with Aβ40 peptide and keep Aβ40 in a randomized and non-fibrillary conformation. The anti-fibrillogenic property of this tripeptide is also supported by the Aβ42 solubilization assay.

Lys-Val-Asp-Asp-Gln-Asp (SEQ ID NO:22), a hexapeptide, when incubated with Aβ40 under physiological conditions shows an increase at time t=0 in the amount of β-sheet content as is evident by the CD spectrum. Incubation of this hexapeptide with Aβ40 for 24 hours shows a dramatic increase in β-sheet content of the Aβ40 and clearly indicates the ability of this hexapeptide to strongly interact with Aβ40 peptide and organize it into a β-sheet conformation. Electron microscopy of the mixture failed to show any fibrils indicating that this particular compound is in fact an anti-fibrillogenic compound with regard to Aβ. *In vitro* results with NBD and thioflavin-T based fluorescence assays confirm this finding. It is the understanding of the inventors that this interesting observation will lead to a greater understanding of fibrillogenesis of Aβ40 and Aβ42 peptides and as a result, will provide important information for the design of potent anti-fibrillogenic compounds for Aβ, other amyloidotic peptides such as AA, AL and IAPP for the treatment of diseases such as Alzheimer's, Type II Diabetes and amyloidosis related disorders. The same principle can also be applied to the design of peptide type compounds for the inhibition of binding of various chemokines to the cell surface as well as inhibition of self assembly and cellular adherence of prion proteins.

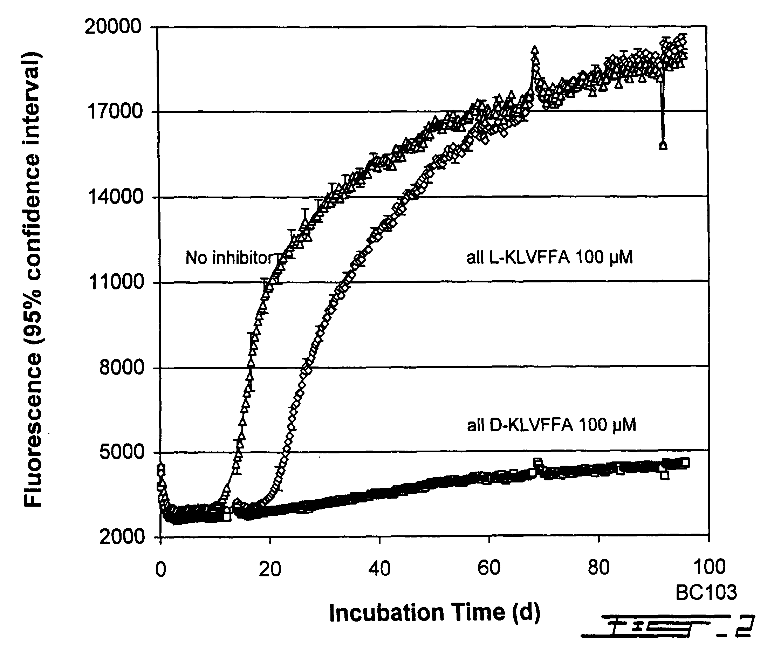

The results illustrated in Fig. 2 show that all [D]-Lys-Leu-Val-Phe-Phe-Ala (SEQ. ID NO: 3) is a more potent inhibitor of Aβ (1-40) assembly in the thioflavin T fluorescence assay than is all [L] -Lys-Leu-Val-Phe-Phe-Ala. Since the naturally occurring Aβ (1-40) used in these experiments was the all-[L] amino acid version, these results indicate that an inhibitor peptide works best when containing amino acids of the opposite chirality.

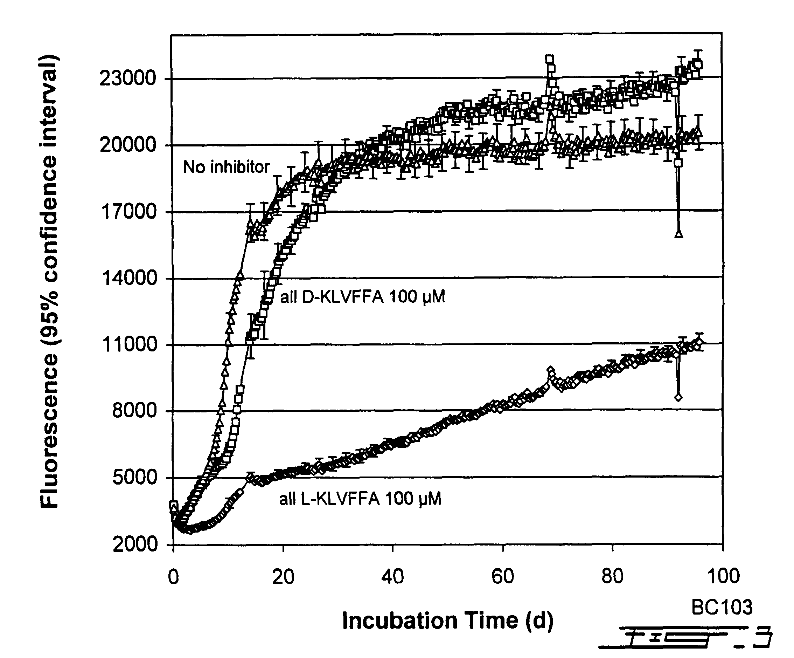

Fig. 3 demonstrates that the same rule of opposite chirality illustrated in Fig. 2 applies for the assembly of Aβ (1-40) synthesized using amino acids of the [D] type. In this experiment all-[L]-Lys-Leu-Val-Phe-Phe-Ala (SEQ. ID NO: 3) is a more potent inhibitor in the all-[D]-Aβ (1-40) assembly reaction than all-[D]-Lys-Leu-Val-Phe-Phe-Ala. This result confirms that peptides of opposite chirality are better inhibitors.

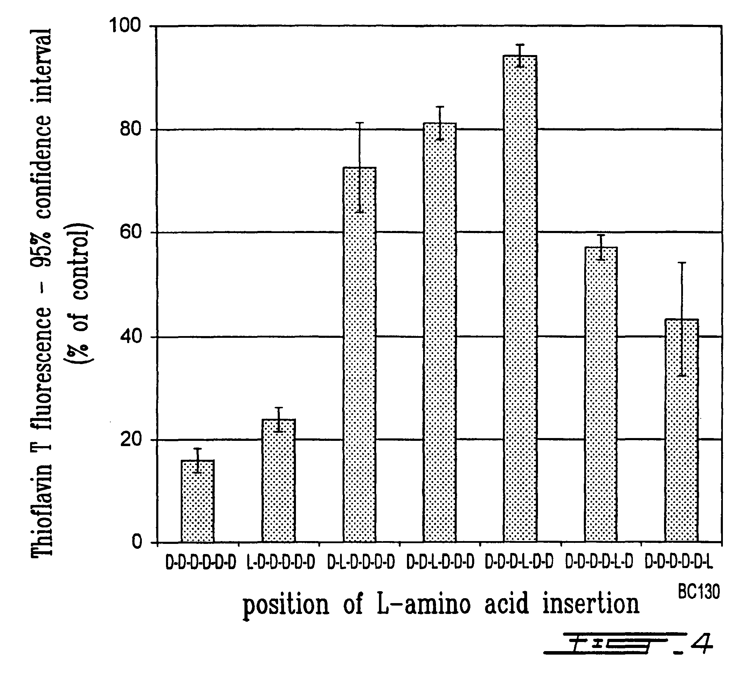

Fig. 4 illustrates the inhibition of Aβ (1-40) fibril formation by all-[D]-Lys-Leu-Val-Phe-Phe-Ala (20μM) with or without single substitutions of [L]-amino acids in the thioflavin T fluorescence assay. In this experiment the ability of the all-[D]-Lys-Leu-Val-Phe-Phe-Ala peptide to inhibit Aβ (1-40) fibril formation, measured as percentage of thioflavin T fluorescence in the absence of peptide (control), was compared to [D]-Lys-Leu-Val-Phe-Phe-Ala peptides with single [L]-amino acid replacements. None of the mixed chirality Lys-Leu-Val-Phe-Phe-Ala peptides were more potent than the original all-[D] peptide. This result demonstrates that [D]-amino acids are more potent inhibitors of Aβ (1-40) fibrillogenesis than [L]-amino acids.

However as seen in Fig. 4 some peptides with single [L] substitutions do retain inhibitory activity. In particular peptides in which the [D] isomer of the Lys, the second Phe and the Ala are substituted with the [L]-isomers retain inhibitory activity. The substitutions, which reduce inhibitory activity the most, are the Leu, the Val and the first Phe, indicating that these residues contribute the most to the potency of the [D]-peptide. From Fig. 4, it is apparent that peptides with mixed chirality or with at least one [D]-substituted amino acid are also inhibitors, although not as potent as the all-[D] peptide.

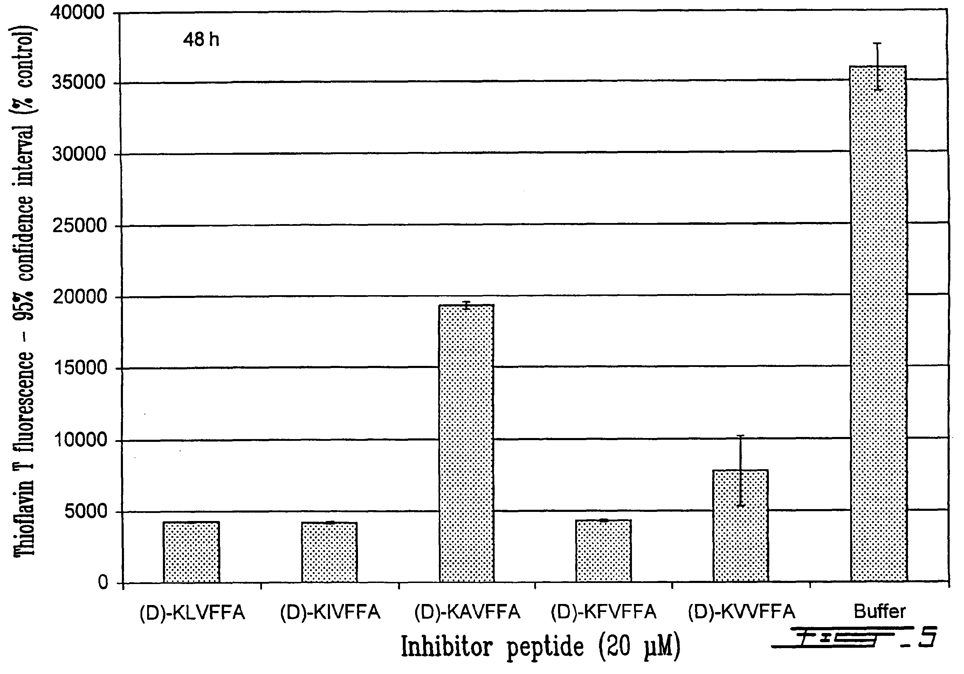

Fig. 5 illustrates the inhibition of Aβ (1-40) fibril formation in the thioflavin T fluorescence assay by all- [D] -Lys-Leu-Val-Phe-Phe-Ala (20μM), with or without replacement of the leucine by other hydrophobic amino acids. In this experiment all the peptides tested retained some inhibitory activity. This result demonstrates that the leucine residue is not critical for inhibition of Aβ fibril formation in the all-[D] peptide. These results illustrated in Fig. 5 were non-obvious and unexpected in light of a prior publication which identified the Leucine residue as critical in an all- [L] version of the peptide (Tjernberg LO et al., J. Biol. Chem. 271:8545, 1996).

## Cytoprotection

The experimental work performed leading to this invention also included comparing the ability of [D] and [L] stereoisomers of the peptides of the present invention to show cytoprotective activity, i.e. to protect cells from Aβ toxicity.

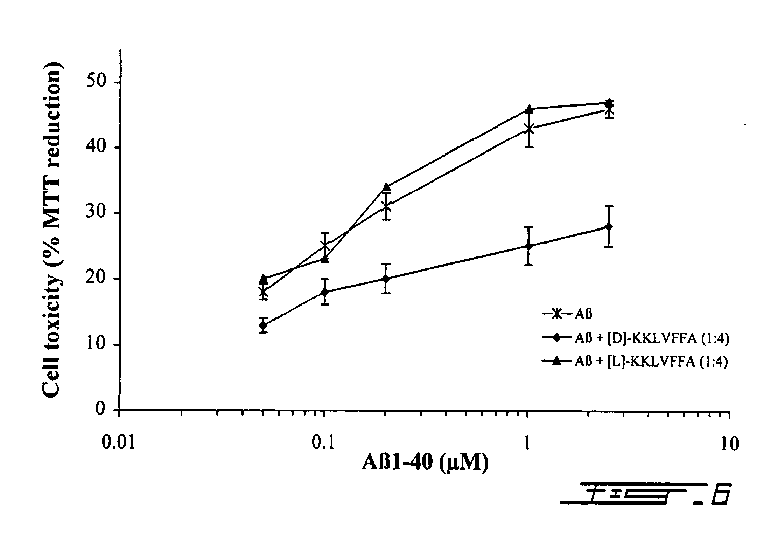

Figure 6 uses the MTT assay on SH-SY5Y cells.

## Protocol

A SH-SY5Y human neuroblast cell line (American Type Culture Collection, cat. CRL-2266) is cultured according to technical specifications. Monomerized Aβ (1-40) is prepared using trifluoroacetic acid and hexafluoroisopropanol, in the same way already described for the thioflavin T fluorescence assay. Monomerized Aβ at various concentrations in TANA buffer (0.02 M TRIS base pH 7.4, 0.02M acetate, 0.15 M NaCl) is added to 100 $\mu$M test peptide and the mixture is incubated for 24 hours at 37°C with agitation, in order to allow polymerization to occur. Cells are adhered to a 96-well microplate for 2 hours at 37°C and 5% $CO_2$ before the Aβ-peptide mixture, or buffer alone (control), is added. The microplate is gently agitated and incubated for 20-24 hours at 37°C and 5% $CO_2$. Cell viability is determined by a MTT-based colorimetric assay. The MTT assay (Boehringer Mannheim, Cell Proliferation Kit 1) is based on the principle that the yellow tetrazolium salt MTT is cleaved in metabolically-active cells to produce purple formazan crystals. The formazan crystals are solubilized and the resulting colored solution is quantified using a scanning multiwell spectrophotometer (ELISA reader, Absorbance $A_{560}$ nm). Cellular toxicity was calculated as follows:

$$\text{Toxicity (\%)} = 100 - \frac{(\text{O.D. sample} - \text{O.D. Blank})}{(\text{O.D. Control} - \text{O.D. Blank})}.$$

Figure 6 shows the neurotoxicity of Aβ (1-40) in the absence or presence of various peptides of the present invention. In this experiment the all-[D]-Lys-Lys-Leu-Val-Phe-Phe-Ala (SEQ. ID NO: 2) peptide is a more potent inhibitor of Aβ neurotoxicity than the all-[L]-Lys-Lys-Leu-Val-Phe-Phe-Ala peptide in the cytoprotection assay.

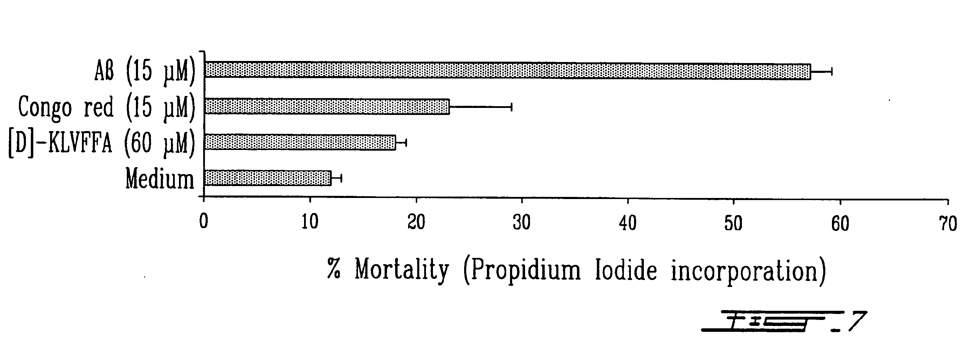

Figure 7 uses the propidium iodide assay on primary cortical neurons. Briefly, fetal rat primary cortical neurons are isolated and cultured according to Durkin, J.P. et al., J. Neurochem., 66:951-962, 1996. Neurons are plated in a 48 well microplate. 7 days after plating the neuronal culture media is supplemented with B27 (Life Technologies, Data sheet form No. 3755). A mixture of Aβ and test peptide is added to the cortical neurons for 3 days at 37°C and 5% CO2.
Cell viability is then visually assessed as the proportion of phase-bright cells that exclude propidium iodide, since only dead cells take up propidium iodide.
Figure 7 shows the potent cytoprotective activity of all-[D]-Lys-Leu-Val-Phe-Phe-Ala (SEQ ID NO:3). This experiment shows the potent cytoprotective activity of all-[D]-Lys-Leu-Val-Phe-Phe-Ala compared to Congo red, which is a known cytoprotective agent and compared to the absence of any cytoprotective agent (Aβ alone).

## Claims

1. An antifibrillogenic agent for inhibiting amyloidosis and/or for cytoprotection, which comprises a peptide consisting of Formula I and consisting of all [D]-amino acids, or a retro-isomer of a peptide consisting of Formula I and consisting of all [D]-amino acids:

$$Xaa_1-Xaa_2-Xaa_3-Xaa_4 \qquad \text{(Formula I)}$$

wherein,

$Xaa_1$ is selected from the group consisting of Lys and $Xaa_5$ -Lys-;
$Xaa_5$ is selected from the group consisting of Lys, His-Gln, His-His-Gln-, Val-His-His-Gln-, Glu-Val-His-His-Gln-, Asp-Asp-Asp-, and Gln-;
$Xaa_2$ is any amino acid;
$Xaa_3$ is Val; and
$Xaa_4$ is selected from the group consisting of Phe, Phe-$NH_2$, Phe-Phe, Phe-Phe-$MH_2$, Phe-Phe-Ala, Phe-Phe-Ala-$NH_2$, Phe-Phe-Ala-Gln, and Phe-Phe-Ala-Gln-$NH_2$.

2. The antifibrillogenic agent of claim 1, wherein $Xaa_2$ is a hydrophobic amino acid residue.

3. The antifibrillogenic agent of claim 1, wherein $Xaa_2$ is an amino acid residue selected from the group consisting of Leu, Ile, Ala, Val, and Phe.

4. The antifibrillogenic agent of claim 1, wherein said retro-isomer is selected from the group consisting of:

Ala-Phe-Phe-Val-Leu-Lys        (SEQ ID NO:5);

and

Ala-Phe-Phe-Val-Leu-Lys-NH$_2$     (SEQ ID NO:13).

5. The antifibrillogenic agent of any one of claims 1 to 4, wherein said peptide of Formula I is selected from the group consisting of:

Lys-Ile-Val-Phe-Phe-Ala     (SEQ ID NO:1);

Lys-Lys-Leu-Val-Phe-Phe-Ala     (SEQ ID NO:2);

Lys-Leu-Val-Phe-Phe-Ala     (SEQ ID NO:3);

Lys-Phe-Val-Phe-Phe-Ala     (SEQ ID NO:4);

Lys-Leu-Val-Phe     (SEQ ID NO:6);

Lys-Ala-Val-Phe-Phe-Ala     (SEQ ID NO:7);

Lys-Leu-Val-Phe-Phe     (SEQ ID NO:8);

Lys-Val-Val-Phe-Phe-Ala     (SEQ ID NO:9);

Lys-Ile-Val-Phe-Phe-Ala-NH$_2$     (SEQ ID NO:10);

Lys-Leu-Val-Phe-Phe-Ala-NH$_2$     (SEQ ID NO: 11);

Lys-Phe-Val-Phe-Phe-Ala-NH$_2$     (SEQ ID NO: 12);

Lys-Leu-Val-Phe-NH$_2$     (SEQ ID NO: 14);

Lys-Ala-Val-Phe-Phe-Ala-NH$_2$     (SEQ ID NO:15);

Lys-Leu-Val-Phe-Phe-NH$_2$     (SEQ ID NO:16);

Lys-Val-Val-Phe-Phe-Ala-NH$_2$     (SEQ ID NO:17);

Lys-Leu-Val-Phe-Phe-Ala-Gln     (SEQ ID NO:18);

Lys-Leu-Val-Phe-Phe-Ala-Gln-NH$_2$     (SEQ ID NO:19);

His-His-Gln-Lys-Leu-Val-Phe-Phe-Ala-NH$_2$     (SEQ ID NO:20);

and

Gln-Lys-Leu-Val-Phe-Phe-NH$_2$     (SEQ ID NO:24).

6. The antifibrillogenic agent of claim 1, wherein the peptide of Formula I is a peptide of SEQ ID NO:2 or SEQ ID NO:3.

7. An antifibrillogenic agent according to any of claims 1-6, formulated in a composition comprising a therapeutically effective amount of said antifibrillogenic agent and a pharmaceutically effective carrier.

8. The antifibrillogenic agent of any one of claims 1 to 6, wherein said peptide of Formula I or retro-isomer thereof is conjugated to a label for *in vivo* imaging.

9. The antifibrillogenic agent of claim 8, wherein said label is [99m]Tc.

10. The antifibrillogenic agent of claim 8 or 9, for use in the manufacture of a compound for *in vivo* imaging of amyloid deposits in a mammal.

11. The antifibrillogenic agent of any one of claims 1 to 6, for use in the treatment of amyloid-β disorders in a patient.

12. The use of an antifibrillogenic agent according to any of claims 1 to 6 in the manufacture of a medicament for the treatment of amyloid-β disorders in a patient.

13. The use according to claim 12, wherein said medicament comprises a therapeutically effective amount of a peptide of Formula I or a retro-isomer thereof, and a pharmaceutically acceptable carrier.

14. The use according to either of claims 12 or 13. wherein said amyloid-β disorder is a neurodegenerative disorder.

15. The use according to claim 14, wherein said neurodegenerative disorder is Alzheimer's disease.

**Patentansprüche**

1. Antifibrillogenes Mittel zum Hemmen von Amyloidose und/oder zum Zellschutz, welches ein Peptid, bestehend aus Formel I und bestehend aus gänzlich [D]-Aminosäuren, oder ein Retroisomer eines Peptids, bestehend aus Formel I und bestehend aus gänzlich [D]-Aminosäuren, umfaßt:

$$Xaa_1\text{-}Xaa_2\text{-}Xaa_3\text{-}Xaa_4 \qquad \text{(Formel I)}$$

wobei

$Xaa_1$ aus der Gruppe, bestehend aus Lys und $Xaa_5$-Lys-, ausgewählt ist;
$Xaa_5$ aus der Gruppe, bestehend aus Lys, His-Gln-, His-His-Gln-, Val-His-His-Gln-, Glu-Val-His-His-Gln-, Asp-Asp-Asp- und Gln-, ausgewählt ist;
$Xaa_2$ eine Aminosäure ist;
$Xaa_3$ Val ist; und
$Xaa_4$ aus der Gruppe, bestehend aus Phe, Phe-$NH_2$, Phe-Phe, Phe-Phe-$NH_2$, Phe-Phe-Ala, Phe-Phe-Ala-$NH_2$, Phe-Phe-Ala-Gln und Phe-Phe-Ala-Gln-$NH_2$, ausgewählt ist.

2. Antifibrillogenes Mittel nach Anspruch 1, wobei $Xaa_2$ ein hydrophober Aminosäurerest ist.

3. Antifibrillogenes Mittel nach Anspruch 1, wobei $Xaa_2$ ein Aminosäurerest, ausgewählt aus der Gruppe, bestehend aus Leu, Ile, Ala, Val und Phe, ist.

4. Antifibrillogenes Mittel nach Anspruch 1, wobei das Retroisomer aus der Gruppe, bestehend aus:

Ala-Phe-Phe-Val-Leu-Lys    (SEQ ID NO: 5);

und

Ala-Phe-Phe-Val-Leu-Lys-$NH_2$    (SEQ ID NO: 13),

ausgewählt ist.

5. Antifibrillogenes Mittel nach einem der Ansprüche 1 bis 4, wobei das Peptid der Formel I aus der Gruppe, bestehend aus:

Lys-Ile-Val-Phe-Phe-Ala    (SEQ ID NO: 1);

Lys-Lys-Leu-Val-Phe-Phe-Ala    (SEQ ID NO: 2);

Lys-Leu-Val-Phe-Phe-Ala    (SEQ ID NO: 3);

Lys-Phe-Val-Phe-Phe-Ala    (SEQ ID NO: 4);

Lys-Leu-Val-Phe   (SEQ ID NO: 6);

Lys-Ala-Val-Phe-Phe-Ala   (SEQ ID NO: 7);

Lys-Leu-Val-Phe-Phe   (SEQ ID NO: 8);

Lys-Val-Val-Phe-Phe-Ala   (SEQ ID NO: 9);

Lys-Ile-Val-Phe-Phe-Ala-$NH_2$   (SEQ ID NO: 10);

Lys-Leu-Val-Phe-Phe-Ala-$NH_2$   (SEQ ID NO: 11);

Lys-Phe-Val-Phe-Phe-Ala-$NH_2$   (SEQ ID NO: 12);

Lys-Leu-Val-Phe-$NH_2$   (SEQ ID NO: 14);

Lys-Ala-Val-Phe-Phe-Ala-$NH_2$   (SEQ ID NO: 15);

Lys-Leu-Val-Phe-Phe-$NH_2$   (SEQ ID NO: 16);

Lys-Val-Val-Phe-Phe-Ala-$NH_2$   (SEQ ID NO: 17);

Lys-Leu-Val-Phe-Phe-Ala-Gln   (SEQ ID NO: 18);

Lys-Leu-Val-Phe-Phe-Ala-Gln-$NH_2$   (SEQ ID NO: 19);

His-His-Gln-Lys-Leu-Val-Phe-Phe-Ala-$NH_2$   (SEQ ID NO: 20);

und

Gln-Lys-Leu-Val-Phe-Phe-$NH_2$   (SEQ ID NO: 24),

ausgewählt ist.

6. Antifibrillogenes Mittel nach Anspruch 1, wobei das Peptid der Formel I ein Peptid von SEQ ID NO: 2 oder SEQ ID NO: 3 ist.

7. Antifibrillogenes Mittel gemäß einem der Ansprüche 1-6, formuliert in einer Zusammensetzung, umfassend eine therapeutisch wirksame Menge des antifibrillogenen Mittels und einen pharmazeutisch wirksamen Träger.

8. Antifibrillogenes Mittel nach einem der Ansprüche 1 bis 6, wobei das Peptid der Formel I oder ein Retroisomer davon mit einer Markierung zur *in-vivo*-Abbildung konjugiert ist.

9. Antifibrillogenes Mittel nach Anspruch 8, wobei die Markierung $^{99m}$Tc ist.

10. Antifibrillogenes Mittel nach Anspruch 8 oder 9 zur Verwendung bei der Herstellung einer Verbindung zur *in-vivo*-Abbildung von Amyloid-Ablagerungen bei einem Säuger.

11. Antifibrillogenes Mittel nach einem der Ansprüche 1 bis 6 zur Verwendung bei der Behandlung von Amyloid-β-Erkrankungen bei einem Patienten.

12. Verwendung eines antifibrillogenen Mittels gemäß einem der Ansprüche 1 bis 6 bei der Herstellung eines Medikaments für die Behandlung von Amyloid-β-Erkrankungen bei einem Patienten.

13. Verwendung gemäß Anspruch 12, wobei das Medikament eine therapeutisch wirksame Menge eines Peptids der Formel I oder eines Retroisomers davon und einen pharmazeutisch verträglichen Träger umfaßt.

**14.** Verwendung gemäß einem der Ansprüche 12 oder 13, wobei die Amyloid-β-Erkrankung eine neurodegenerative Erkrankung ist.

**15.** Verwendung gemäß Anspruch 14, wobei die neurodegenerative Erkrankung die Alzheimer-Krankheit ist.

**Revendications**

**1.** Agent antifibrillogène pour inhiber l'amyloïdose et/ou pour la cytoprotection, lequel comprend un peptide consistant en la Formule 1 et consistant en acides aminés tous [D], ou un isomère rétro d'un peptide consistant en la Formule 1 et consistant en acides aminés tous [D]:

$$Xaa_1\text{-}Xaa_2\text{-}Xaa_3\text{-}Xaa_4 \qquad (Formule\ I)$$

dans lequel,

$Xaa_1$ est sélectionné parmi le groupe consistant en Lys ou en $Xaa_5$-Lys-;
$Xaa_5$ est sélectionné parmi le groupe consistant en Lys, His-Gln-; His-His-Gln-, Val-His-His-Gln-, Glu-Val-His-His-Gln-, Asp-Asp-Asp- et Gln-;
$Xaa_2$ est un acide aminé quelconque;
$Xaa_3$ est Val; et
$Xaa_4$ est sélectionné parmi le groupe consistant en Phe, Phe-NH$_2$, Phe-Phe, Phe-Phe-NH$_2$, Phe-Phe-Ala, Phe-Phe-Ala-NH$_2$, Phe-Phe-Ala-Gln et Phe-Phe-Ala-Gln-NH$_2$.

**2.** L'agent antifibrillogène de la revendication 1, dans lequel $Xaa_2$ est un résidu d'acide aminé hydrophobe.

**3.** L'agent antifibrillogène de la revendication 1, dans lequel $Xaa_2$ est un résidu d'acide aminé sélectionné parmi le groupe consistant en Leu, Ile, Ala, Val et Phe.

**4.** L'agent antifibrillogène de la revendication 1, dans lequel ledit isomère rétro est sélectionné parmi le groupe consistant en:

$$Ala\text{-}Phe\text{-}Phe\text{-}Val\text{-}Leu\text{-}Lys \qquad (SEQ\ ID\ NO:\ 5);$$

et

$$Ala\text{-}Phe\text{-}Phe\text{-}Val\text{-}Leu\text{-}Lys\text{-}NH_2 \qquad (SEQ\ ID\ NO:\ 13).$$

**5.** L'agent antifibrillogène de l'une quelconque des revendications 1 à 4, dans lequel ledit peptide de la Formule I est sélectionné parmi le groupe consistant en:

$$Lys\text{-}Ile\text{-}Val\text{-}Phe\text{-}Phe\text{-}Ala \qquad (SEQ\ ID\ NO:\ 1);$$

$$Lys\text{-}Lys\text{-}Leu\text{-}Val\text{-}Phe\text{-}Phe\text{-}Ala \qquad (SEQ\ ID\ NO:\ 2);$$

$$Lys\text{-}Leu\text{-}Val\text{-}Phe\text{-}Phe\text{-}Ala \qquad (SEQ\ ID\ NO:\ 3);$$

$$Lys\text{-}Phe\text{-}Val\text{-}Phe\text{-}Phe\text{-}Ala \qquad (SEQ\ ID\ NO:\ 4);$$

$$Lys\text{-}Leu\text{-}Val\text{-}Phe \qquad (SEQ\ ID\ NO:\ 6);$$

$$Lys\text{-}Ala\text{-}Val\text{-}Phe\text{-}Phe\text{-}Ala \qquad (SEQ\ ID\ NO:\ 7);$$

$$Lys\text{-}Leu\text{-}Val\text{-}Phe\text{-}Phe \qquad (SEQ\ ID\ NO:\ 8);$$

$$Lys\text{-}Val\text{-}Val\text{-}Phe\text{-}Phe\text{-}Ala \qquad (SEQ\ ID\ NO:\ 9);$$

$$Lys\text{-}Ile\text{-}Val\text{-}Phe\text{-}Phe\text{-}Ala\text{-}NH_2 \qquad (SEQ\ ID\ NO:\ 10);$$

Lys-Leu-Val-Phe-Phe-Ala-NH$_2$      (SEQ ID NO: 11);

Lys-Phe-Val-Phe-Phe-Ala-NH$_2$      (SEQ ID NO: 12);

Lys-Leu-Val-Phe-NH$_2$      (SEQ ID NO: 14);

Lys-Ala-Val-Phe-Phe-Ala-NH$_2$      (SEQ ID NO: 15);

Lys-Leu-Val-Phe-Phe-NH$_2$      (SEQ ID NO: 16);

Lys-Val-Val-Phe-Phe-Ala-NH$_2$      (SEQ ID NO: 17);

Lys-Leu-Val-Phe-Phe-Ala-Gln      (SEQ ID NO: 18);

Lys-Leu-Val-Phe-Phe-Ala-Gln-NH$_2$      (SEQ ID NO: 19);

His-His-Gln-Lys-Leu-Val-Phe-Phe-Ala-NH$_2$      (SEQ ID NO: 20);

et

Gln-Lys-Leu-Val-Phe-Phe-NH$_2$      (SEQ ID NO: 24).

**6.** L'agent antifibrillogène de la revendication 1, dans lequel le peptide de la Formule 1 est un peptide de la SEQ ID NO: 2 ou de la SEQ ID NO: 3

**7.** Agent antifibrillogène selon l'une quelconque des revendications 1-6, formulé dans une composition comprenant une quantité thérapeutiquement efficace dudit agent antifibrillogène et un excipient pharmaceutiquement efficace.

**8.** L'agent antifibrillogène de l'une quelconque des revendications 1 à 6, dans lequel ledit peptide de la Formule I ou isomère rétro de celui-ci est conjugué à un marqueur pour imagerie *in* vivo.

**9.** L'agent antifibrillogène de la revendication 8, dans lequel ledit marqueur est $^{99m}$Tc.

**10.** L'agent antifibrillogène de la revendication 8 ou 9, à utiliser dans la fabrication d'un composé destiné à l'imagerie *in vivo* de dépôts de substance amyloïde chez un mammifère.

**11.** L'agent antifibrillogène de l'une quelconque des revendications 1 à 6, à utiliser dans le traitement de troubles causés par la médiation d'amyloïde β chez un patient.

**12.** L'utilisation d'un agent antifibrillogène selon l'une quelconque des revendications 1 à 6, dans la fabrication d'un médicament pour le traitement de troubles causés par la médiation d'amyloïde β chez un patient.

**13.** L'utilisation selon la revendication 12, dans laquelle ledit médicament comprend une quantité thérapeutiquement efficace d'un peptide de la Formule I ou d'un isomère rétro de celui-ci et un excipient pharmaceutiquement acceptable.

**14.** L'utilisation selon la revendication 12 ou la revendication 13, dans laquelle ledit trouble causé par la médiation d'amyloïde β est un trouble neurodégénératif.

**15.** L'utilisation selon la revendication 14, dans laquelle ledit trouble neurodégénératif est la maladie d'Alzheimer.

# Protein - Protein Interaction:
## Targetted Sites

GAG binding
site/microglia
activation

13-16    16-21    ——→ ß-sheet

DAEFRHDSGYEVHHQKLVFFAEDVGSNKGAIIGLMVGGVVIA

10-16

Cell surface adherence

40-42

C-terminal

EP 1 173 480 B1

*Fig. 1*

Fluorescence (95% confidence interval)

No inhibitor    all L-KLVFFA 100 μM

all D-KLVFFA 100 μM

Incubation Time (d)

BC103

*FIG._2*

EP 1 173 480 B1

Fluorescence (95% confidence interval)

Incubation Time (d)

BC103

Fig. 3

No inhibitor

all D-KLVFFA 100 µM

all L-KLVFFA 100 µM

position of L-amino acid insertion

BC130

FIG. 4

EP 1 173 480 B1

*Fig. 5*

EP 1 173 480 B1

EP 1 173 480 B1

% Mortality (Propidium Iodide incorporation)

Aß (15 µM)
Congo red (15 µM)
[D]-KLVFFA (60 µM)
Medium

0    10    20    30    40    50    60    70

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

## Patent documents cited in the description

- WO 9808868 A

- US 60132592 B

## Non-patent literature cited in the description

- **TJERNBERG L.O. et al.** *Journal Biol.Chem.,* 1997, vol. 272 (19), 12601-12605
- Biologically Active Peptides: Design, Synthesis, and Utilization. Technomic Publishing Company Inc, 1993, vol. 1, 35-3
- *Biochemistry,* 1972, vol. 11, 1726-1732
- Proteoglycans and amyloid fibrillogenesis: The nature and origin of amyloid fibrils. **KISILEVSKY, R. et al.** CIBA Foundation Symposium. Wiley, 1997, 58-72
- **GUPTA-BANSAL, R. et al.** *The Journal of Biological Chemistry,* 1995, vol. 270, 18666-18671
- **GIULIAN, D. et al.** *The Journal of Biological Chemistry,* 1998, vol. 273 (45), 29719-29726
- **GIULIAN, D. et al.** *The Journal of Neuroscience,* 1996, vol. 16 (19), 6021-6037

- **HILBRICH, C. et al.** *J. Mol. Biol.,* 1992, vol. 228, 460-473
- **TJERNBERG, L.O. et al.** Controlling Amyloid β-Peptide Fibril Formation with Protease-stable Ligands. *J. Biol. Chem.,* 1997, vol. 272, 12602
- **LEVINE III, H.** *Protein Science,* 1993, vol. 2, 404-410
- **NAIKI, H. et al.** *Lab. Invest.,* 1996, vol. 74, 374-383
- **LEVINE III, H.** *Amyloid: Int. J. Exp. Clin Invest.,* 1995, vol. 2, 1.6
- **BRONFMAN, P.C. et al.** *Neuroscience Lett.,* 1995, vol. 218, 201-203
- **TJERNBERG LO et al.** *J. Biol. Chem.,* 1996, vol. 271, 8545
- **DURKIN, J.P. et al.** *J. Neurochem.,* 1996, vol. 66, 951-962